Europäisches Patentamt

European Patent Office

Office européen des brevets

(19)

(11) Veröffentlichungsnummer : **0 222 984**
**B1**

(12) # EUROPÄISCHE PATENTSCHRIFT

(45) Veröffentlichungstag der Patentschrift :
28.09.88

(21) Anmeldenummer : 86111313.2

(22) Anmeldetag : 16.08.86

(51) Int. Cl.⁴ : **C 07 C 31/135**, C 07 C 29/19,
C 07 C 29/136

(54) **Verfahren zur Herstellung von Cyclohexylverbindungen.**

(30) Priorität : 19.10.85 DE 3537228

(43) Veröffentlichungstag der Anmeldung :
27.05.87 Patentblatt 87/22

(45) Bekanntmachung des Hinweises auf die Patenterteilung : 28.09.88 Patentblatt 88/39

(84) Benannte Vertragsstaaten :
AT BE CH DE FR GB IT LI NL SE

(56) Entgegenhaltungen :
DE-A- 1 443 888
DE-A- 1 542 380
DE-A- 2 605 610
DE-A- 2 804 075
GB-A- 1 545 563
US-A- 3 366 695
PATENTS ABSTRACTS OF JAPAN, unexamined applications, C Sektion, Vol. 3, Nr. 27, 7. März 1979. THE PATENT OFFICE JAPANESE GOVERNMENT, Seite 5 C 39

(73) Patentinhaber : HÜLS AKTIENGESELLSCHAFT
Patentabteilung / PB 15 - Postfach 13 20
D-4370 Marl 1 (DE)

(72) Erfinder : Otte, Werner, Dr.
Eichenstück 55
D-4270 Dorsten 11 (DE)
Erfinder : Nehring, Rudolf, Dr.
Griesheimer Strasse 12
D-4370 Marl (DE)

EP 0 222 984 B1

## Beschreibung

Die Erfindung betrifft ein Verfahren zur Hydrierung von benzylischen Verbindungen, insbesondere Actophenon, zu den entsprechenden Cyclohexyl-Verbindungen in Gegenwart eines Ru-Katalysators.

Das erfindungsgemäße Verfahren zielt darauf ab, die Cyclohexyl-Verbindungen in einfacher und wirtschaftlicher Arbeitsweise in hoher Reinheit und Ausbeute herzustellen.

1-Cyclohexylethanol-1 ist als Ausgangssubstanz für die Herstellung von Vinylcyclohexan von Interesse, das seinen Einsatz in der Polymerchemie findet.

Die Hydrierung benzylischer Verbindungen zu den entsprechenden gesättigten Cyclohexylverbindungen mit Hilfe von Ru-, Rh- und Pd-Katalysatoren ist bekannt, jedoch sind die bekannten Verfahren durch niedrige Ausbeuten gekennzeichnet, besonders durch die Bildung von Cycloparaffinen.

Zur Behebung dieser Schwierigkeiten werden daher überwiegend Katalysatorkombinationen, wie in SU-PS 733 710 (Ru + Cr auf $Al_2O_3$), SU-PS 405 323 (Rh + Ru auf $Al_2O_3$), SU-PS 488 703 (Ni + Cr), Tetrahedron Letters, 17, S. 1663-1664, (1967) (Pd, Pd + Rh, Pd + Ru auf Kohle) oder Zusätze von z. B. Alkali wie in US-PS 3 366 695 beschrieben, eingesetzt. Auch der Zusatz von Säuren ist bekannt. Diese Maßnahmen führen zwar zu einer Ausbeuteverbesserung, dafür sind aber zusätzliche Verfahrensschritte erforderlich. Nach Zh. Prikl. Khim. 42 (11), S. 2613-2614 (1969) sollen Rh-Kontakte besonders geeignet sein. Pd-Kontakte werden insbesondere bei höheren Drücken wenig wirksam.

Überraschend wurde gefunden, daß bei höheren Drücken die Hydrierung von Acetophenon zu 1-Cyclohexan-ethanol-1 an handelsüblichen (z. B. Engelhard) Ru-Kontakten, wobei das Ru auf inerten Trägern, wie $Al_2O_3$, oder z. B. $SiO_2$ etc., aufgebracht ist, in nahezu quantitativer Ausbeute gelingt.

Gegenstand der Erfindung ist daher ein Verfahren zur Herstellung von Cyclohexyl-Verbindungen durch katalytische Hydrierung von benzylischen Verbindungen dadurch gekennzeichnet, daß man die benzylischen Verbindungen in Gegenwart eines Ru-Trägerkatalysators in der Sumpf- oder Rieselphase bei einem Wasserstoffdruck von 200-350 bar und Temperaturen von 80 bis etwa 160 °C hydriert.

Das Verfahren kann sowohl kontinuierlich als auch diskontinuierlich durchgeführt werden, wobei in der Regel das kontinuierliche bevorzugt wird. Die für das erfindungsgemäße Verfahren notwendigen Apparaturen gehören zum Stand der Technik, z. B. Rührautoklaven oder Reaktionsrohr. Es ist möglich, das erfindungsgemäße Verfahren in der Sumpfphase oder in der Rieselphase zu betreiben. Bei der diskont. Arbeitsweise wird üblich in der Sumpfphase in einem Autoklaven in Gegenwart von pulverförmigem Katalysator gearbeitet.

Das erfindungsgemäße Verfahren kann besonders vorteilhaft kontinuierlich durchgeführt werden. Dies erfolgt in üblicher Weise mit stückig (Kugeln, Pellets, etc.) angeordnetem Katalysator, z. B. nach dem Rieselphasenprinzip, wobei das Ausgangsprodukt flüssig oder teilweise dampfförmig über den im Reaktionsrohr befindlichen Katalysator strömt, während der Wasserstoff im Gleich- oder Gegenstrom durch das Reaktionsrohr geleitet wird. Hierbei ist es vorteilhaft, überschüssigen Wasserstoff im Kreis zu führen.

Im allgemeinen wird das erfindunggemäße Verfahren ohne Zusatz eines Lösemittels durchgeführt, jedoch ist die Anwendung eines Lösemittels nicht schädlich.

Ferner wird das erfindungsgemäße Verfahren bei einem Druck oberhalb 100 bar, insbesondere oberhalb 200 oder 250 bar bis etwa 320 bar und Temperaturen zwischen 80 °C und etwa 160 °C, insbesondere zwischen 100 und etwa 130 °C durchgeführt. Die LHSV beträgt etwa 0,25.

Der beim erfindungsgemäßen Verfahren eingesetzte Ru-Katalysator ist ein handelssüblicher (z. B. Engelhard), wobei das Ru auf inerten Trägern wie z. B. $Al_2O_3$ oder $SiO_2$, in Konzentrationen von 0,1 bis 1 %, vorzugsweise von 0,3-1 % und insbesondere 0,5 % aufgebracht ist.

Die besonderen Vorteile des erfindungsgemäßen Verfahrens gegenüber bekannten Hydrierverfahren bestehen darin, daß mit geringeren Mengen an Ruthenium als es dem Stand der Technik entspricht [US-PS 3 366 695, Zh, Prikl., Khim., 39 (11), S. 2599 bis 2601, (1966)] gearbeitet oder auf den Einsatz von Edelmetallgemischen [SU-PS 733 710, SU-PS 405 323, Tetrahedron Lett., 17, S. 1663-1664 (1967)] verzichtet werden kann. Außerdem sind die Edelmetallkosten beim Ru deutlich geringer als beim bisher bevorzugten Rhodium [Chem. Lett. 5, S. 603-606 (1982), SU-PS 448 703 Zh. Prikl. Chim. 10, S. 2398-2400 (1969) und dto. 11, S. 2613-2614 (1969)].

Zudem kommt man mit etwa bzw. weniger als der 10 fach kleineren Edelmetallkonzentration als üblich zu sehr guten Ergebnissen, d. h. 2,5 bis 5 g Ru-Metall pro t Acetophenon.

Das erfindungsgemäße Verfahren führt in wirtschaftlicher Weise und in hoher Ausbeute zu Cyclohexyl-Verbindungen.

Folgende Beispiele sollen das erfindungsgemäße Verfahren illustrieren :

### Beispiel 1

Ein 400 ml Rohrreaktor, gefüllt mit einem 0,5 % Ru/$Al_2O_3$-Kontakt (Engelhard) wird mit 100 ml/h (ca. 0,83 Mol) Acetophenon und 400 Nl/h $H_2$ (ca. 18 Mol) bei 300 bar Gesamtdruck und 110 °C beschickt. Der Hydrieraustrag hat folgende Zusammensetzung (nach GC) :

**0 222 984**

| | |
|---|---|
| Vorläufe | 0,3 % |
| Cyclohexylethan | 1,7 % |
| Zwischenläufe | 0,2 % |
| Acetophenon | — |
| 1-Cyclohexylethanol | 96,4 % |
| Nachläufe | 1,4 % |

Das eingesetzte Acetophenon hatte eine Reinheit von 96,7 %.

Nach einer Laufzeit von 2 200 h wurde die Versuchsperiode abgebrochen, wobei praktisch kein Selektivitäts- und Aktivitätsverlust auftrat.

## Beispiel 2

Aufführung wie in Beispiel 1, jedoch wird als Zulauf eine Lösung aus gleichen Gewichtsteilen Acetophenon und 1-Cyclohexylethanol eingesetzt. Der Hydrieraustrag setzt sich wie folgt zusammen :

| | |
|---|---|
| Vorläufe | 0,5 % |
| Cyclohexylethan | 1,9 % |
| Zwischenläufe | 0,3 % |
| Acetophenon | — |
| 1-Cyclohexylethanol | 95,8 % |
| Nachläufe | 1,5 % |

Der Zulauf bestand aus 48,4 % Acetophenon und 48,2 % 1-Cyclohexylethanol, der Rest sind Vor-, Zwischen- und Nachläufe.

## Beispiel 3

Ausführung wie im Beispiel 1, jedoch wird die Temperatur auf 90 °C gesenkt. Der Hydrieraustrag hat folgende Zusammensetzung :

| | |
|---|---|
| Vorläufe | 0,1 % |
| Cyclohexylethan | 0,8 % |
| Zwischenläufe | 0,5 % |
| Acetophenon | 6,1 % |
| 1-Cyclohexylethanol | 91,0 % |
| Nachläufe | 1,5 % |

Das eingesetzte Acetophenon hatte eine Reinheit von 97,4 %.

## Beispiel 4

Ausführung wie in Beispiel 1, jedoch wird die Temperatur auf 150 °C erhöht. Der Hydrieraustrag setzt sich wie folgt zusammen :

| | |
|---|---|
| Vorläufe | 1,2 % |
| Cyclohexylethan | 4,1 % |
| Zwischenläufe | 0,3 % |
| Acetophenon | — |
| 1-Cyclohexylethanol | 92,9 % |
| Nachläufe | 1,5 % |

Das eingesetzte Acetophenon hatte eine Reinheit von 97,4 %.

## Beispiel 5

Ausführung wie in Beispiel 1, jedoch wird der Druck auf 200 bar erniedrigt. Der Hydrieraustrag hat folgende Zusammensetzung :

| | |
|---|---|
| Vorläufe | 0,2 % |
| Cyclohexylethan | 1,2 % |
| Zwischenläufe | 0,2 % |
| Acetophenon | 0,9 % |
| 1-Cyclohexylethanol | 96,1 % |
| Nachläufe | 1,4 % |

Das eingesetzte Acetophenon hatte eine Reinheit von 97,4 %.

Vergleichsbeispiel 1

Ausführung wie in Beispiel 1, jedoch wird ein Katalysator mit 0,5 % Pd auf Al₂O₃ (Engelhard) eingesetzt. Der Hydrieraustrag hat folgende Zusammensetzung :

| Vorläufe | 0,4 % |
| Acetophenon | Spuren |
| Ethylcyclohexan | 65,2 % |
| Zwischenläufe | 2,6 % |
| 1-Cyclohexylethanol | 29,5 % |
| Nachläufe | 2,3 % |

Vergleichsbeispiel 2

Ausführung wie in Beispiel 1, jedoch wird ein Katalysator mit 0,5 % Pd auf Al₂O₃ (Engelhard) eingesetzt, der Druck auf 15 bar und die Temperatur auf 90 °C gesenkt. Der Hydrieraustrag hat folgende Zusammensetzung :

| Vorläufe | 0,2 % |
| Acetophenon | 0,8 % |
| Ethylbenzol | 34,2 % |
| Zwischenläufe | 2,0 % |
| Methylbenzylalkohol | 61,1 % |
| Nachläufe | 1,7 % |

**Patentansprüche**

1. Verfahren zur Herstellung von Cyclohexyl-Verbindungen durch katalytische Hydrierung von benzylischen Verbindungen dadurch gekennzeichnet, daß man die benzylischen Verbindungen in Gegenwart eines Ru-Trägerkatalysators in der Slumpf- oder Rieselphase bei einem Wasserstoffdruck von 200-350 bar und Temperaturen von 80 bis etwa 160 °C hydriert.

2. Verfahren nach Anspruch 1, dadurch gekennzeichnet, daß man als benzylische Verbindung Acetophenon einsetzt.

3. Verfahren nach den Ansprüchen 1 bis 2, dadurch gekennzeichnet, daß der Katalysator 0,1 bis 1 Gewichtsprozent Ru enthält.

4. Verfahren nach Anspruch 3, dadurch gekennzeichnet, daß der Katalysator 0,5 Gewichtsprozent Ru enthält.

5. Verfahren nach den Ansprüchen 1 bis 4, dadurch gekennzeichnet, daß man als Träger Al₂O₃ verwendet.

6. Verfahren nach den Ansprüchen 1 bis 5, dadurch gekennzeichnet, daß man bei einem Wasserstoffdruck von 250 bis 320 bar hydriert.

7. Verfahren nach den Ansprüchen 1 bis 6, dadurch gekennzeichnet, daß man bei Temperaturen von 100 bis 130 °C, hydriert.

**Claims**

1. Process for preparing cyclohexyl compounds by catalytic hydrogenation of benzylic compounds, characterized in that the benzylic compound is hydrogenated in the presence of a Ru-carrier catalyst in a bottom phase or in a trickling phase at a hydrogen pressure of 200-350 bar and a temperature of 80 to about 160 °C.

2. Process according to Claim 1 characterized in that acetophenone is employed as the benzylic compound.

3. Process according to Claim 1 or 2 characterized in that the catalyst contains 0.1 to 1.0 % by weight of Ru.

4. Process according to Claim 3 characterized in that the catalyst contains 0.5 % by weight of Ru.

5. Process according to any one of Claims 1 to 4 characterized in that Al₂O₃ is employed as carrier.

6. Process according to any one of Claims 1 to 5 characterized in that hydrogenation is performed at a hydrogen pressure of 250 to 320 bar.

7. Process according to any one of Claims 1 to 6 characterized in that hydrogenation is performed at

**0 222 984**

a temperature of 100 to 130 °C.

**Revendications**

1. Procédé d'obtention de composés cyclohexaniques par hydrogénation catalytique de composés benzyliques, caractérisé en ce que l'on hydrogène les composés benzyliques en présence d'un catalyseur support à base de Ruthénium en phase puisard ou de ruissellement sous une pression d'hydrogène de 200 à 350 bars et à des températures allant de 80 à environ 160 °C.

2. Procédé selon la revendication 1, caractérisé en ce que l'on met en œuvre en tant que composé benzylique, l'acétophénone.

3. Procédé selon les revendications 1 à 2, caractérisé en ce que le catalyseur renferme de 0,1 à 1 % en poids de Ruthénium.

4. Procédé selon la revendication 3, caractérisé en ce que le catalyseur renferme 0,5 % en poids de Ruthénium.

5. Procédé selon les revendications 1 à 4, caractérisé en ce qu'on utilise en tant que support de l'alumine $Al_2O_3$.

6. Procédé selon les revendications 1 à 5, caractérisé en ce qu'on hydrogène à une pression d'hydrogène de 250 à 320 bars.

7. Procédé selon les revendications 1 à 6, caractérisé en ce que l'on hydrogène à des températures allant de 100 à 130 °C.

5